(19) Europäisches Patentamt
European Patent Office
Office européen des brevets

(11) **EP 3 952 763 B1**

(12) **EUROPEAN PATENT SPECIFICATION**

(45) Date of publication and mention
of the grant of the patent:
**05.11.2025  Bulletin 2025/45**

(21) Application number: **20722838.8**

(22) Date of filing: **07.04.2020**

(51) International Patent Classification (IPC):
*A61B 17/3207* (2006.01)   *G05B 19/404* (2006.01)
*A61B 17/00* (2006.01)   *A61B 90/00* (2016.01)
*G05B 19/25* (2006.01)

(52) Cooperative Patent Classification (CPC):
**A61B 17/320758; G05B 19/256;** A61B 2017/00017;
A61B 2017/00398; A61B 2090/031; A61B 2090/066;
A61B 2090/08021; G05B 2219/45117

(86) International application number:
**PCT/US2020/027079**

(87) International publication number:
**WO 2020/210238 (15.10.2020 Gazette 2020/42)**

(54) **ATHERECTOMY SYSTEM WITH EXCESS TORQUE PROTECTION**

ATHEREKTOMIESYSTEM MIT SCHUTZ VOR ÜBERMÄSSIGEM DREHMOMENT

SYSTÈME D'ATHÉRECTOMIE À PROTECTION CONTRE UNE TORSION ÉLEVÉE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.04.2019  US 201962830990 P**

(43) Date of publication of application:
**16.02.2022  Bulletin 2022/07**

(73) Proprietor: **Boston Scientific Scimed, Inc.
Maple Grove, MN 55311 (US)**

(72) Inventors:
• **CARLSON, Corydon**
**Stillwater, Minnesota 55082 (US)**
• **OHRT, Gary Thomas**
**Corcoran, Minnesota 55374 (US)**
• **STRELOW, Wade Robert**
**Maple Grove, Minnesota 55369 (US)**

(74) Representative: **Vossius & Partner
Patentanwälte Rechtsanwälte mbB
Siebertstraße 3
81675 München (DE)**

(56) References cited:
WO-A1-2019/118522   WO-A2-00/56230
WO-A2-2013/158849   US-A- 4 679 557

**Description**

CROSS-REFERENCE TO RELATED APPLICATIONS

This application claims the benefit of priority under 35 U.S.C. §119 to U.S. Provisional Application Serial No. 62/830,990 filed April 8, 2019.

TECHNICAL FIELD

The present disclosure pertains to medical devices, and methods for manufacturing and using medical devices. More particularly, the disclosure is directed to devices and methods for removing occlusive material from a body lumen. Further, the disclosure is directed to an atherectomy device for forming a passageway through an occlusion of a body lumen, such as a blood vessel.

BACKGROUND

Many patients suffer from occluded arteries and other blood vessels which restrict blood flow. Occlusions can be partial occlusions that reduce blood flow through the occluded portion of a blood vessel or total occlusions (e.g., chronic total occlusions) that substantially block blood flow through the occluded blood vessel. In some cases a stent may be placed in the area of a treated occlusion. However, restenosis may occur in the stent, further occluding the vessel and restricting blood flow. Revascularization techniques include using a variety of devices to pass through the occlusion to create or enlarge an opening through the occlusion. Atherectomy is one technique in which a catheter having a cutting element thereon is advanced through the occlusion to form or enlarge a pathway through the occlusion.
US 4679557 A discloses an electromechanical system which may be used in conjunction with spinning catheters, such as those which have differential cutting burrs, includes an electric prime mover which attaches to an advancer assembly. The advancer assembly has an off-axis drive and apparatus for loading a catheter unit, which is driven through a catheter engagement coupling. The catheter assembly can also include one or two lumens with access ports at the advancer assembly, whereby medicines may be injected or pressure readings may be obtained from the vessel.
A need remains for alternative atherectomy devices to facilitate crossing an occlusion.

SUMMARY

The invention is defined by independent claim 1. The dependent claims define preferred embodiments.
This disclosure provides design, material, manufacturing method, and use alternatives for medical devices. For example, an atherectomy system includes an atherectomy burr and a drive mechanism that is adapted to rotatably actuate the atherectomy burr. A controller is adapted to regulate operation of the drive mechanism and to calculate an estimated load torque at the atherectomy burr based upon at least one of an angular velocity of the atherectomy system and an angular acceleration of the atherectomy system. The controller is further adapted to stop or reverse the drive mechanism when the estimated load torque at the atherectomy burr exceeds a torque threshold.
Alternatively or additionally, the controller may be adapted to determine an angular position of the atherectomy system.
Alternatively or additionally, the controller may be adapted to determine an angular velocity of the atherectomy system by determining a first derivative with respect to time of the angular position.
Alternatively or additionally, the controller may be adapted to determine an angular acceleration of the atherectomy system by determining a second derivative with respect to time of the angular position.
Alternatively or additionally, the controller may be adapted to calculate the estimated load torque $T_{load}$ at the atherectomy burr in accordance with equation (1):

$$T_{load} = K_T * i - C_D * \dot{\theta} - I * \ddot{\theta} \qquad (1),$$

where

$K_T$ is a torque constant for the drive motor;
$i$ is a drive motor current;
$C_D$ is a coefficient of friction value;
$\dot{\theta}$ is the angular velocity of the atherectomy system;
$I$ is an inertia of the atherectomy system; and

$\ddot{\theta}$ is the angular acceleration of the atherectomy system.

Alternatively or additionally, i may be a measured or calculated value.

Alternatively or additionally, $C_D$ may be a constant.

Alternatively or additionally, $C_D$ may be a calculated value.

Alternatively or additionally, the drive mechanism may include a drive cable that is coupled with the atherectomy burr and a drive motor that is adapted to rotate the drive cable.

As another example, an atherectomy system includes a drive mechanism that is adapted to rotatably actuate an atherectomy burr and a controller that is adapted to regulate operation of the drive mechanism. The controller is adapted to calculate an estimated load torque at the atherectomy burr $T_{load}$ in accordance with equation (2):

$$T_{load} = T_{motor} - T_{drag} - I * \ddot{\theta} \qquad (2),$$

where

$T_{motor}$ is an estimated motor torque for the drive motor;
$T_{drag}$ is an estimated drag torque for the drive mechanism;
$I$ is a system inertia value; and
$\ddot{\theta}$ is an angular acceleration value. The controller is further adapted to stop or reverse the drive mechanism when $T_{load}$ exceeds a torque threshold.

Alternatively or additionally, $T_{motor}$ may be calculated by the controller in accordance with equation (3):

$$T_{motor} = K_T * i \qquad (3),$$

where

$K_T$ is a torque constant for the drive motor; and
$i$ is a drive motor current.

Alternatively or additionally, $i$ may be a measured or calculated value.

Alternatively or additionally, $T_{drag}$ may be calculated by the controller in accordance with equation (4):

$$T_{drag} = C_D * \dot{\theta} \qquad (4),$$

where

$C_D$ is a coefficient of friction value; and
$\dot{\theta}$ is an angular velocity value.

Alternatively or additionally, $C_D$ may be a constant.

Alternatively or additionally, $C_D$ may be a time varying value.

Alternatively or additionally, when running at steady state, $T_{motor}$ is substantially equal to $T_{drag}$, and thus at steady state $T_{load}$ may be calculated by the controller in accordance with equation (5):

$$T_{load} = -I * \ddot{\theta} \qquad (5).$$

Alternatively or additionally, the drive mechanism may include a drive cable that is coupled with the atherectomy burr and a drive motor that is adapted to rotate the drive cable.

As another example, an atherectomy system includes a drive mechanism that is adapted to rotatably actuate an atherectomy burr and a controller that is adapted to regulate operation of the drive mechanism. The controller is adapted to stop or reverse the drive mechanism when an estimated torque value $T_{load}$ exceeds a torque threshold. When the atherectomy system is at steady state, the controller is adapted to calculate $T_{load}$ in accordance with equation (5):

$$T_{load} = -I * \ddot{\theta} \qquad (5),$$

where

I is an inertia of the atherectomy system; and
$\ddot{\theta}$ is the angular acceleration of the atherectomy system; and

wherein when the atherectomy system is accelerating, the controller is adapted to calculate $T_{load}$ in accordance with equation (1):

$$T_{load} = K_T * i - C_D * \dot{\theta} - I * \ddot{\theta} \qquad (1),$$

where

$K_T$ is a torque constant for the drive motor;
i is a drive motor current;
$C_D$ is a coefficient of friction value; and
$\dot{\theta}$ is the angular velocity of the atherectomy system.

Alternatively or additionally, the drive mechanism may be adapted to accelerate the atherectomy burr to full speed in less than 2 seconds.

Alternatively or additionally, the drive mechanism may include a drive motor having a power rating of at least about 60 watts.

The above summary of some embodiments is not intended to describe each disclosed embodiment or every implementation of the present disclosure. The Figures, and Detailed Description, which follow, more particularly exemplify these embodiments.

BRIEF DESCRIPTION OF THE DRAWINGS

The invention may be more completely understood in consideration of the following detailed description of various embodiments of the invention in connection with the accompanying drawings, in which:

FIG. 1 is a schematic block diagram of an example atherectomy system;
FIG. 2 is a schematic block diagram of an example atherectomy system;
FIG. 3 is a schematic block diagram of an example atherectomy system;
FIG. 4 is a schematic block diagram of an example atherectomy system;
FIG. 5 is a schematic block diagram of an example atherectomy system; and
FIG. 6 is a schematic diagram of an example PID controller usable in the example atherectomy systems of FIGS. 1 through 5.

While the disclosure is amenable to various modifications and alternative forms, specifics thereof have been shown by way of example in the drawings and will be described in detail. It should be understood, however, that the intention is not to limit the disclosure to the particular embodiments described. The invention is defined by the claims.

DETAILED DESCRIPTION

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The drawings, which are not necessarily to scale, depict illustrative embodiments and are not intended to limit the scope of the invention.

Many patients suffer from occluded arteries, other blood vessels, and/or occluded ducts or other body lumens which may restrict bodily fluid (e.g. blood, bile, etc.) flow. Occlusions can be partial occlusions that reduce blood flow through the occluded portion of a blood vessel or total occlusions (e.g., chronic total occlusions) that substantially block blood flow through the occluded blood vessel. Revascularization techniques include using a variety of devices to pass through the occlusion to create or enlarge an opening through the occlusion. Atherectomy is one technique in which a catheter having a cutting element thereon is advanced through the occlusion to form or enlarge a pathway through the occlusion. Ideally, the cutting element excises the occlusion without damaging the surrounding vessel wall and/or a previously implanted stent where restenosis has occurred. However, in some instances the cutting element may be manipulated and/or advanced such that it contacts the vessel wall and/or the stent. Therefore, it may be desirable to utilize materials and/or design an atherectomy device that can excise an occlusion without damaging the surrounding vessel and/or a previously implanted stent where restenosis has occurred. Additionally, it may be desirable that a cutting element be useful in removing hard occlusive material, such as calcified material, as well as softer occlusive material. The methods and systems disclosed herein may be designed to overcome at least some of the limitations of previous atherectomy devices while effectively excising occlusive material. For example, some of the devices and methods disclosed herein may include cutting elements with unique cutting surface geometries and/or designs.

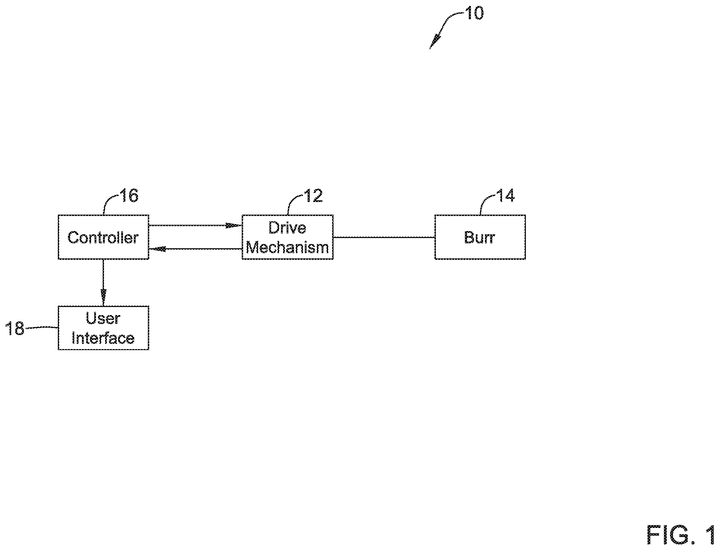

FIG. 1 is a schematic block diagram of an example atherectomy system 10 that includes a drive mechanism 12 that is adapted to rotatably actuate an atherectomy burr 14. The atherectomy system 10 includes a controller 16 that is adapted to regulate operation of the drive mechanism 12. In some cases, the atherectomy system 10 may include a user interface 18 that may be operably coupled to the controller 16 such that the controller 16 is able to display information regarding the performance of the drive mechanism 12. This information may, for example, include one or more of an instantaneous speed of the drive mechanism 12, an instantaneous torque being experienced by the atherectomy burr 14, and the like. In some instances, the atherectomy system 10 may not include the user interface 18. In some cases, the atherectomy burr 14 may also be referred to as being or including a cutting head or a cutting member, and these terms may be used interchangeably.

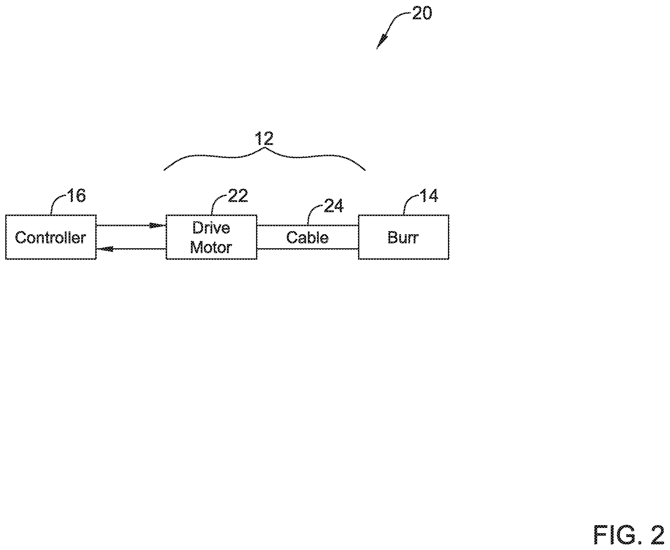

FIG. 2 is a schematic block diagram of an example atherectomy system 20 in which the drive mechanism 12 may include a drive motor 22 and a drive cable 24 that is operably coupled with the drive motor 22 as well as the atherectomy burr 14. In some cases, features of the atherectomy system 20 may be combined with features of the atherectomy system 10. In some cases, the atherectomy system 20 may also include a handle (not shown).

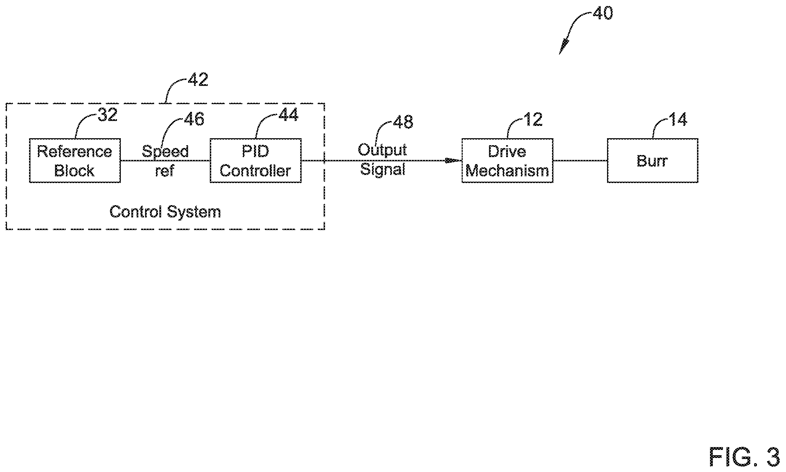

FIG. 3 is a schematic block diagram of an example atherectomy system 40 that includes a control system 42 that is adapted to regulate operation of the drive mechanism 12 in order to rotatably actuate the atherectomy burr 14. In some cases, features of the atherectomy system 40 may be combined with one or more of the atherectomy system 10 and the atherectomy system 20. The control system 42 may include a reference block 32 as well as a Proportional Integral Derivative (PID) controller 44 that is operably coupled to the reference block 32. In some cases, the reference block 32 may determine a speed reference 46 that is selectable between a nominal value, a negative value and zero. In some instances, the PID controller 44 may be further adapted to add an offset value to the speed reference 46 received from the reference block 32, although in some cases, the reference block 32 may add the offset value. The PID controller 44 may be further adapted to provide a reduction in motor speed of the drive mechanism 12 that is greater than what would otherwise normally occur in response to an increasing torque experienced at the atherectomy burr 14.

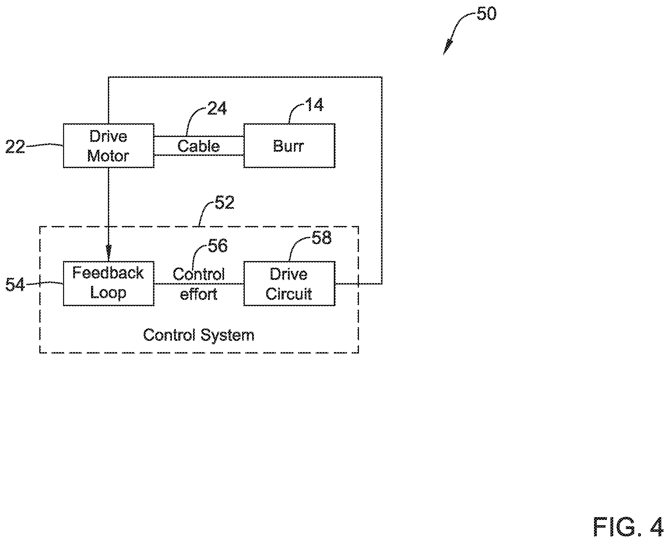

FIG. 4 is a schematic block diagram of an example atherectomy system 50 that includes a control system 52 that is adapted to regulate operation of the drive motor 22 in order to rotatably actuate the atherectomy burr 14. In some cases, features of the atherectomy system 50 may be combined with one or more of the atherectomy system 10, the atherectomy system 20 or the atherectomy system 40. The control system 52 is operably coupled to the drive motor 22 and includes a feedback loop 54 that is adapted to monitor performance of the drive motor 22 and to output a control effort signal 56. A drive circuit 58 is adapted to receive the control effort signal 56 and to regulate operation of the drive motor 22 in accordance with the control effort signal 56.

In some cases, the feedback loop 54 may include a reference block for determining a speed reference and a Proportional Integral Derivative (PID) controller that is operably coupled to the reference block for receiving the speed reference, the PID controller adapted to utilize the speed reference, a Proportional (P) gain value, an Integral (I) gain value and a Derivative (D) gain value in determining the control effort signal. In some cases, the feedback loop 54 may be adapted to add an offset value to a reference signal provided to the reference loop 54 in order to accurately hold speed of the drive motor 22 during a no-load situation. In some instances, for example if the atherectomy burr 14 becomes stuck, the control system 52 may be further adapted to increase the torque provided by the drive motor 22 until a torque threshold is reached for a brief period of time, and to subsequently direct the drive motor 22 to reverse at a slow speed in order to unwind energy in the drive mechanism.

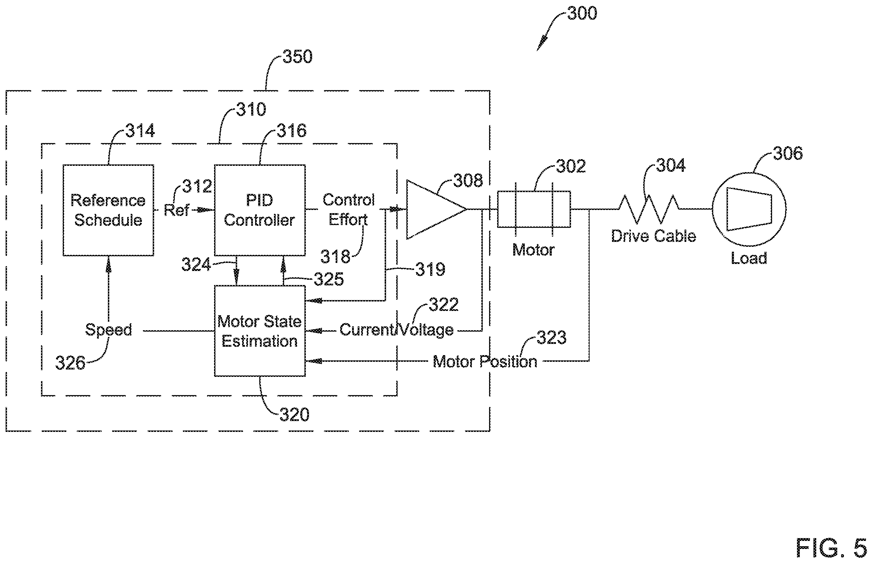

FIG. 5 is a schematic block diagram of an example atherectomy system 300. In some cases, the atherectomy system 300 may be considered as being an example of the atherectomy system 10, 20, 30, 40 or 50. In some instances, features of the atherectomy system 300 may be combined with features of any of the atherectomy systems 10, 20, 30, 40 or 50, for example. The atherectomy system 300 includes a motor 302 that drives a drive cable 304 which itself engages a load 306. The load 306 represents an atherectomy burr, for example. The motor 302 is controlled by a drive circuitry 308 which may be considered as being an example of or otherwise incorporated into the drive module 22 and/or the control system 16, for example. In some cases, the motor 302 may be sized, relative to the weight and other dimensions of the atherectomy system 300, to be capable of accelerating the atherectomy burr to full speed in less than 3 seconds, or in some cases in less than 2 seconds. As an example, the motor 302 may be rated for at least 60 watts. In a particular example, the motor 302 may be rated for about 80 watts. These are just examples.

The drive circuitry 308 receives an input from a feedback portion 310. In some cases, the feedback portion 310 begins with a reference input 312 from a reference schedule block 314, which provides the reference input 312 to a PID controller 316. In some cases, the reference schedule block 314 may be configured to accept additional inputs, such as from a user and/or from additional sensors not illustrated. As an example, if the device has been running for too long of a period of time, the reference schedule block 314 may reduce the speed reference in order to prevent overheating. A PID controller is a controller that includes a (P) proportional portion, an (I) integral portion and a (D) derivative portion. The PID controller 316 outputs a control effort value or reference current 318 to the drive circuitry 308. A motor state estimation block 320 receives a current/voltage signal 322 and a motor position signal 323 from the drive circuitry 308 and receives state feedback 324 from the PID controller 316. The motor state estimation block 320 provides a state feedback signal 325 back to the PID controller 316.

The motor state estimation block 320 outputs a speed value 326 back to the reference schedule block 314. While the feedback from the motor state estimation block 320 to the reference schedule block 314 is shown as being a speed value, in some cases the feedback may additionally or alternatively include one or more of position, torque, voltage or current, and in some cases may include the derivative or integral of any of these values. In some cases, the motor state estimation block 320 may instead receive a signal 323 that represents speed, instead of position (as illustrated). The motor position signal 323 may be an indication of relative rotational position of an output shaft of the motor 302, and thus an indication of relative rotational position of the load 306, which if tracked over time may provide an indication of speed.

In some cases, the drive circuitry 308 and the feedback loop 310 may in combination be considered as forming a controller 350 that is adapted to determine an estimated torque at the atherectomy burr (the load 306 as shown in FIG. 5). The controller 350 may be considered as being an example of the controller 16 (FIG. 1). In some cases, the controller 350 may be considered as including only some elements of the drive circuitry 308 and the feedback loop 310. In some instances, some of the features and functions of the controller 350 may take place in the motor state estimation block 320. It will be appreciated that while FIG. 5 shows various components as standalone components, in some cases the functions of one or more of the components may actually be spread between separate mechanical components. In some instances, the functions of one or more of the components may be combined into one or more mechanical components.

If the estimated torque at the load 306 becomes too high, this may be an indication that the burr is getting stuck. In order to protect against possible damage to the drive cable 304, and to protect against possible injury to the patient, the atherectomy system 300 may be adapted to stop or even reverse operation of the atherectomy system 300 if the estimated torque meets or exceeds a predetermined torque threshold. It will be appreciated that the actual value of the predetermined torque threshold may vary, depending on the mechanics of the atherectomy system 300, but may be set at a level low enough to prevent damage and injury, but not set so low as to engender too many false alarms caused by minor and/or temporary torque increases that are not caused by the load 306 becoming stuck. For example, the instantaneous torque may vary by small amounts as the atherectomy system 300 progresses through the patient's vasculature.

Accordingly, the controller 350 may be adapted to calculate an estimated torque at the load 306 and to compare the estimated torque at the load 306 to the torque threshold. If the estimated torque meets or exceeds the torque threshold, the atherectomy system 300 may stop or even reverse the drive mechanism (the drive motor 302 and the drive cable 304, for example). In some instances, the atherectomy system 300 may be adapted to calculate an estimated torque at the load 306 based upon at least one of an angular velocity of the atherectomy system 300 and an angular acceleration of the atherectomy system 300.

In some instances, the controller 350 may be adapted to determine an angular position of the atherectomy system 300. This may mean determining an angular position of the motor 302, or that of the cable 304. It will be appreciated that the controller 350 may be adapted to determine an angular velocity of the atherectomy system 300 by determining a first derivative with respect to time of the angular position. The controller 350 may be adapted to determine an angular acceleration of the atherectomy system 300 by determining a second derivative with respect to time of the angular position. In some instances, for example, the controller 350 may be adapted to calculate an estimated torque at the load 306, indicated by $T_{load}$, in accordance with equation (1):

$$T_{load} = K_T * i - C_D * \dot{\theta} - I * \ddot{\theta} \qquad (1),$$

where

$K_T$ is a torque constant for the drive motor;
$i$ is a drive motor current;
$C_D$ is a coefficient of friction value;
$\dot{\theta}$ is the angular velocity of the atherectomy system 300;
$I$ is an inertia of the atherectomy system 300; and
$\ddot{\theta}$ is the angular acceleration of the atherectomy system 300.

In some cases, the drive motor current i may be a measured or calculated value. In some cases, the drive motor current i may be estimated within the motor state estimation block 320. For example, the reference current 318 may be fed into the motor state estimation block 320 via a path 319, and the motor state estimation block 320 may predict the drive motor current *i* more rapidly than the drive motor current *i* could be measured. In some instances, the coefficient of friction $C_D$ may be a constant. In some cases, $C_D$ may be a calculated value or even a time-varying value. In some cases, $C_D$ may be a factor of one or more of an amount of current being commanded, system speed, and the age (total run time of the system). The controller 350 may calculate $C_D$ based on one or more of these factors, for example. In some cases, the controller 350 may include a lookup table, for example, that provides particular values for $C_D$ for each of a number of rotational speed ranges. This is just an example. $\dot{\theta}$ represents the angular velocity of the atherectomy system 300, and as indicated may be determined by taking a first derivative, with respect to time, of the angular position of the atherectomy system 300. $\ddot{\theta}$ represents the angular acceleration of the atherectomy system 300, and as indicated may be determined by taking a second derivative, with respect to time, of the angular position of the atherectomy system 300. The inertia of the system *I* may be easily calculated based on the mass and geometry of the system.

In some cases, the controller 350 may be adapted to calculate an estimated torque at the load 306 in accordance with equation (2):

$$T_{load} = T_{motor} - T_{drag} - I * \ddot{\theta} \qquad (2),$$

where

$T_{motor}$ is an estimated motor torque for the drive motor 302; and
$T_{drag}$ is an estimated drag torque for the drive mechanism.

In some cases, the controller 350 may be adapted to calculate the estimated motor torque $T_{motor}$ in accordance with equation (3) and may calculate the estimated drag torque $T_{drag}$ is calculated by the controller in accordance with equation (4):

$$T_{motor} = K_T * i \qquad (3).$$

$$T_{drag} = C_D * \dot{\theta} \qquad (4).$$

It will be appreciated that in some cases, that when the atherectomy system 300 is running at steady state, and thus is not accelerating, that $T_{motor}$ may be considered as being substantially equal to $T_{drag}$, and thus at steady state $T_{load}$ may be calculated by the controller 350 in accordance with equation (5):

$$T_{load} = -I * \ddot{\theta} \qquad (5).$$

Accordingly, and in some cases when the atherectomy system 300 is at steady state, the controller 350 may be adapted to calculate $T_{load}$ in accordance with equation (5):

$$T_{load} = -I * \ddot{\theta} \qquad (5)$$

and when the atherectomy system 300 is accelerating, the controller 350 may be adapted to calculate $T_{load}$ in accordance with equation (1):

$$T_{load} = K_T * i - C_D * \dot{\theta} - I * \ddot{\theta} \qquad (1).$$

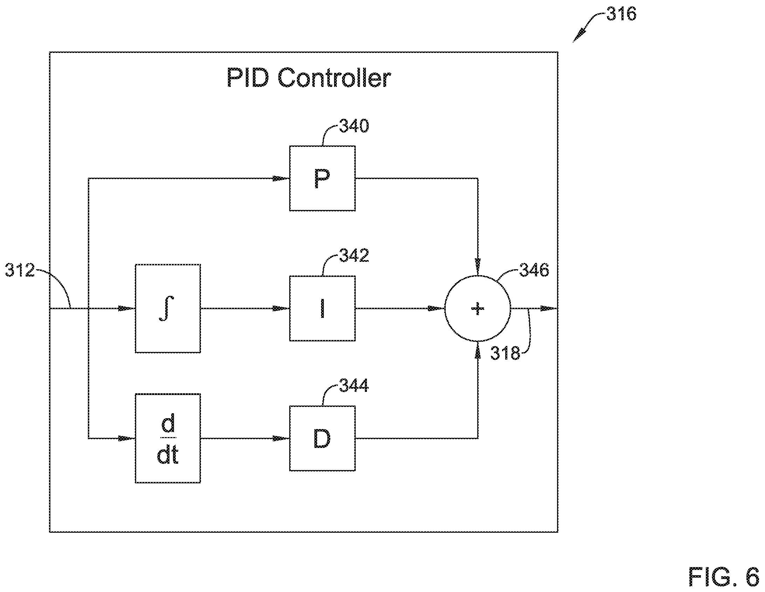

FIG. 6 is a schematic block diagram of the PID controller 316, which may be considered as being an example of the PID controller 44 shown in FIG. 4. An error signal 312, which is representative of an error between a desired value and an actual value, enters the PID controller 316. The PID controller 316 calculates a P term 340, which is proportional to the error. The PID controller 316 calculates an I term 342, which is an integral of the error and a D term 344, which is a derivative of the error. These terms are added together at a summation point 346, resulting in an output of the control effort signal 318.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The invention's scope is, of course, defined in the language in which the appended claims are expressed.

## Claims

1. An atherectomy system (10; 20; 40; 50; 300), comprising:

   an atherectomy burr (14);
   a drive mechanism (12) adapted to rotatably actuate the atherectomy burr (14);
   a controller (16; 42; 52; 350) adapted to regulate operation of the drive mechanism (12);
   the controller (16; 42; 52; 350) adapted to calculate an estimated load torque at the atherectomy burr (14) based upon at least one of an angular velocity of the atherectomy system (10; 20; 40; 50; 300) and an angular acceleration of the atherectomy system (10; 20; 40; 50; 300); and
   the controller (16; 42; 52; 350) further adapted to stop or reverse the drive mechanism (12) when the estimated load torque at the atherectomy burr (14) exceeds a torque threshold.

2. The atherectomy system (10; 20; 40; 50; 300) of claim 1, wherein the controller (16; 42; 52; 350) is adapted to determine an angular position of the atherectomy system (10; 20; 40; 50; 300).

3. The atherectomy system (10; 20; 40; 50; 300) of claim 2, wherein the controller is adapted to determine an angular velocity of the atherectomy system (10; 20; 40; 50; 300) by determining a first derivative with respect to time of the angular position.

4. The atherectomy system (10; 20; 40; 50; 300) of any one of claims 2 or 3, wherein the controller (16; 42; 52; 350) is adapted to determine an angular acceleration of the atherectomy system (10; 20; 40; 50; 300) by determining a second derivative with respect to time of the angular position.

5. The atherectomy system (10; 20; 40; 50; 300) of any one of claims 1 to 4, wherein the controller (16; 42; 52; 350) is adapted to calculate the estimated load torque $T_{load}$ at the atherectomy burr (14) in accordance with equation (1):

$$T_{load} = K_T * i - C_D * \dot{\theta} - I * \ddot{\theta} \qquad (1),$$

   where

   $K_T$ is a torque constant for the drive motor;
   $i$ is a drive motor current;
   $C_D$ is a coefficient of friction value;
   $\dot{\theta}$ is the angular velocity of the atherectomy system;
   $I$ is an inertia of the atherectomy system; and
   $\ddot{\theta}$ is the angular acceleration of the atherectomy system.

6. The atherectomy system (10; 20; 40; 50; 300) of claim 5, wherein $i$ is a measured or calculated value.

7. The atherectomy system (10; 20; 40; 50; 300) of claim 5, wherein $C_D$ is a constant.

8. The atherectomy system (10; 20; 40; 50; 300) of claim 5, wherein $C_D$ is a calculated value.

9. The atherectomy system (10; 20; 40; 50; 300) of any one of claims 1 to 8, wherein the drive mechanism (12) comprises:

a drive cable (24; 304) coupled with the atherectomy burr (14); and
a drive motor (22; 302) adapted to rotate the drive cable (24; 304).

10. The atherectomy system (10; 20; 40; 50; 300) of claim 1, wherein the controller (16; 42; 52; 350) is adapted to calculate an estimated load torque at the atherectomy burr $T_{load}$ in accordance with equation (2):

$$T_{load} = T_{motor} - T_{drag} - I * \ddot{\theta} \qquad (2),$$

where

$T_{motor}$ is an estimated motor torque for the drive motor;
$T_{drag}$ is an estimated drag torque for the drive mechanism;
$I$ is a system inertia value; and
$\ddot{\theta}$ is an angular acceleration value.

11. The atherectomy system (10; 20; 40; 50; 300) of claim 10, wherein $T_{motor}$ is calculated by the controller (16; 42; 52; 350) in accordance with equation (3):

$$T_{motor} = K_T * i \qquad (3),$$

where

$K_T$ is a torque constant for the drive motor; and
$i$ is a drive motor current.

12. The atherectomy system (10; 20; 40; 50; 300) of claim 11, wherein $i$ is a measured or calculated value.

13. The atherectomy system (10; 20; 40; 50; 300) of claim 10, wherein $T_{drag}$ is calculated by the controller (16; 42; 52; 350) in accordance with equation (4):

$$T_{drag} = C_D * \dot{\theta} \qquad (4),$$

where

$C_D$ is a coefficient of friction value; and
$\dot{\theta}$ is an angular velocity value.

14. The atherectomy system (10; 20; 40; 50; 300) of claim 10, wherein the drive mechanism (12) comprises:

a drive cable (24; 304) coupled with the atherectomy burr (14); and
a drive motor (22; 302) adapted to rotate the drive cable (24; 304).

15. The atherectomy system (10; 20; 40; 50; 300) of claim 1,

wherein when the atherectomy system (10; 20; 40; 50; 300) is at steady state, the controller (16; 42; 52; 350) is adapted to calculate the estimated torque value $T_{load}$ in accordance with equation (5):

$$T_{load} = -I * \ddot{\theta} \qquad (5),$$

where
$I$ is an inertia of the atherectomy system; and
$\ddot{\theta}$ is the angular acceleration of the atherectomy system; and
wherein when the atherectomy system (10; 20; 40; 50; 300) is accelerating, the controller (16; 42; 52; 350) is adapted to calculate $T_{load}$ in accordance with equation (1):

$$T_{load} = K_T * i - C_D * \dot{\theta} - I * \ddot{\theta} \qquad (1),$$

where

$K_T$ is a torque constant for the drive motor;

$i$ is a drive motor current;

$C_D$ is a coefficient of friction value;

$\dot{\theta}$ is the angular velocity of the atherectomy system (10; 20; 40; 50; 300); (10; 20; 40; 50; 300).

**Patentansprüche**

1. Atherektomie-System (10; 20; 40; 50; 300), aufweisend:

   einen Atherektomie-Fräser (14);
   einen Antriebsmechanismus (12), der angepasst ist, den Atherektomie-Fräser (14) rotierend zu betätigen;
   eine Steuerung (16; 42; 52; 350), die angepasst ist, einen Betrieb des Antriebsmechanismus (12) zu regeln;
   wobei die Steuerung (16; 42; 52; 350) angepasst ist, auf Basis einer Winkelgeschwindigkeit des Atherektomie-Systems (10; 20; 40; 50; 300) und/oder einer Winkelbeschleunigung des Atherektomie-Systems (10; 20; 40; 50; 300) ein geschätztes Lastdrehmoment an dem Atherektomie-Fräser (14) zu berechnen; und
   die Steuerung (16; 42; 52; 350) ferner angepasst ist, den Antriebsmechanismus (12) zu stoppen oder umzukehren, wenn das geschätzte Lastdrehmoment an dem Atherektomie-Fräser (14) eine Drehmomentschwelle überschreitet.

2. Atherektomie-System (10; 20; 40; 50; 300) nach Anspruch 1, wobei die Steuerung (16; 42; 52; 350) angepasst ist, eine Winkelposition des Atherektomie-Systems (10; 20; 40; 50; 300) zu bestimmen.

3. Atherektomie-System (10; 20; 40; 50; 300) nach Anspruch 2, wobei die Steuerung (16; 42; 52; 350) angepasst ist, durch Bestimmen einer ersten Ableitung der Winkelposition nach der Zeit eine Winkelgeschwindigkeit des Atherektomie-Systems (10; 20; 40; 50; 300) zu bestimmen.

4. Atherektomie-System (10; 20; 40; 50; 300) nach einem der Ansprüche 2 oder 3, wobei die Steuerung (16; 42; 52; 350) angepasst ist, durch Bestimmen einer zweiten Ableitung der Winkelposition nach der Zeit eine Winkelbeschleunigung des Atherektomie-Systems (10; 20; 40; 50; 300) zu bestimmen.

5. Atherektomie-System (10; 20; 40; 50; 300) nach einem der Ansprüche 1 bis 4, wobei die Steuerung (16; 42; 52; 350) angepasst ist, das geschätzte Lastdrehmoment $T_{load}$ an dem Atherektomie-Fräser (14) gemäß Gleichung (1) zu berechnen:

$$T_{load} = K_T * i - C_D * \dot{\theta} - I * \ddot{\theta} \qquad (1),$$

   wobei

   $K_T$ eine Drehmoment-Konstante für den Antriebsmotor ist;
   $i$ ein Antriebsmotorstrom ist;
   $C_D$ ein Reibungskoeffizientenwert ist;
   $\dot{\theta}$ die Winkelgeschwindigkeit des Atherektomie-Systems ist;
   $I$ eine Trägheit des Atherektomie-Systems ist; und
   $\ddot{\theta}$ die Winkelbeschleunigung des Atherektomie-Systems ist.

6. Atherektomie-System (10; 20; 40; 50; 300) nach Anspruch 5, wobei i ein gemessener oder berechneter Wert ist.

7. Atherektomie-System (10; 20; 40; 50; 300) nach Anspruch 5, wobei $C_D$ eine Konstante ist.

8. Atherektomie-System (10; 20; 40; 50; 300) nach Anspruch 5, wobei $C_D$ ein berechneter Wert ist.

9. Atherektomie-System (10; 20; 40; 50; 300) nach einem der Ansprüche 1 bis 8, wobei der Antriebsmechanismus (12) aufweist:

ein Antriebskabel (24; 304), das mit dem Atherektomie-Fräser (14) gekoppelt ist; und
einen Antriebsmotor (22; 302), der angepasst ist, das Antriebskabel (24; 304) zu rotieren.

**10.** Atherektomie-System (10; 20; 40; 50; 300) nach Anspruch 1, wobei die Steuerung (16; 42; 52; 350) angepasst ist, ein geschätztes Lastdrehmoment an dem Atherektomie-Fräser $T_{load}$ gemäß Gleichung (2) zu berechnen:

$$T_{load} = T_{motor} - T_{drag} - I * \ddot{\theta} \qquad (2)$$

wobei

$T_{motor}$ ein geschätztes Motordrehmoment für den Antriebsmotor ist;
$T_{drag}$ ein geschätztes Schleppdrehmoment für den Antriebsmechanismus ist;
$I$ ein Systemträgheitswert ist; und
$\ddot{\theta}$ ein Winkelbeschleunigungswert ist.

**11.** Atherektomie-System (10; 20; 40; 50; 300) nach Anspruch 10, wobei $T_{motor}$ von der Steuerung (16; 42; 52; 350) gemäß Gleichung (3) berechnet wird:

$$T_{motor} = K_T * i \qquad (3)$$

wobei

$K_T$ eine Drehmomentkonstante für den Antriebsmotor ist; und
$i$ ein Antriebsmotorstrom ist.

**12.** Atherektomie-System (10; 20; 40; 50; 300) nach Anspruch 11, wobei $i$ ein gemessener oder berechneter Wert ist.

**13.** Atherektomie-System (10; 20; 40; 50; 300) nach Anspruch 10, wobei $T_{drag}$ von der Steuerung (16; 42; 52; 350) gemäß Gleichung (4) berechnet wird:

$$T_{drag} = C_D * \dot{\theta} \qquad (4)$$

wobei

$C_D$ ein Reibungskoeffizientenwert ist; und
$\dot{\theta}$ ein Winkelgeschwindigkeitswert ist.

**14.** Atherektomie-System (10; 20; 40; 50; 300) nach Anspruch 10, wobei der Antriebsmechanismus (12) aufweist:

ein Antriebskabel (24; 304), das mit dem Atherektomie-Fräser (14) gekoppelt ist; und
einen Antriebsmotor (22; 302), der angepasst ist, das Antriebskabel (24; 304) zu rotieren.

**15.** Atherektomie-System (10; 20; 40; 50; 300) nach Anspruch 1,

wobei, wenn das Atherektomie-System (10; 20; 40; 50; 300) in einem Gleichgewichtszustand ist, die Steuerung (16; 42; 52; 350) angepasst ist, das geschätzte Lastdrehmoment $T_{load}$ gemäß Gleichung (5) zu berechnen:

$$T_{load} = -I * \ddot{\theta} \qquad (5)$$

wobei
$I$ eine Trägheit des Atherektomie-Systems ist; und
$\ddot{\theta}$ die Winkelbeschleunigung des Atherektomie-Systems ist; und
wobei, wenn das Atherektomie-System (10; 20; 40; 50; 300) beschleunigt, die Steuerung (16; 42; 52; 350) angepasst ist, $T_{load}$ gemäß Gleichung (1) zu berechnen:

$$T_{load} = K_T * i - C_D * \dot{\theta} - I * \ddot{\theta} \qquad (1),$$

wobei

$K_T$ eine Drehmoment-Konstante für den Antriebsmotor ist;
$i$ ein Antriebsmotorstrom ist;
$C_D$ ein Reibungskoeffizientenwert ist;
$\dot{\theta}$ die Winkelgeschwindigkeit des Atherektomie-Systems (10; 20; 40; 50; 300) ist.

**Revendications**

1.  Système d'athérectomie (10 ; 20 ; 40 ; 50 ; 300), comprenant :

    une fraise d'athérectomie (14) ;
    un mécanisme d'entraînement (12) adapté pour actionner en rotation la fraise d'athérectomie (14) ;
    un dispositif de commande (16 ; 42 ; 52 ; 350) adapté pour réguler le fonctionnement du mécanisme d'entraînement (12) ;
    le dispositif de commande (16 ; 42 ; 52 ; 350) étant adapté pour calculer un couple de charge estimé au niveau de la fraise d'athérectomie (14) sur la base d'au moins l'une d'une vitesse angulaire du système d'athérectomie (10 ; 20 ; 40 ; 50 ; 300) et d'une accélération angulaire du système d'athérectomie (10 ; 20 ; 40 ; 50 ; 300) ; et
    le dispositif de commande (16 ; 42 ; 52 ; 350) est en outre adapté pour arrêter ou inverser le mécanisme d'entraînement (12) lorsque le couple de charge estimé au niveau de la fraise d'athérectomie (14) dépasse un seuil de couple.

2.  Système d'athérectomie (10 ; 20 ; 40 ; 50 ; 300) selon la revendication 1, dans lequel le dispositif de commande (16 ; 42 ; 52 ; 350) est adapté pour déterminer une position angulaire du système d'athérectomie (10 ; 20 ; 40 ; 50 ; 300).

3.  Système d'athérectomie (10 ; 20 ; 40 ; 50 ; 300) selon la revendication 2, dans lequel le dispositif de commande est adapté pour déterminer une vitesse angulaire du système d'athérectomie (10 ; 20 ; 40 ; 50 ; 300) en déterminant une première dérivée par rapport au temps de la position angulaire.

4.  Système d'athérectomie (10 ; 20 ; 40 ; 50 ; 300) selon l'une quelconque des revendications 2 ou 3, dans lequel le dispositif de commande (16 ; 42 ; 52 ; 350) est adapté pour déterminer une accélération angulaire du système d'athérectomie (10 ; 20 ; 40 ; 50 ; 300) en déterminant une deuxième dérivée par rapport au temps de la position angulaire.

5.  Système d'athérectomie (10 ; 20 ; 40 ; 50 ; 300) selon l'une quelconque des revendications 1 à 4, dans lequel le dispositif de commande (16 ; 42 ; 52 ; 350) est adapté pour calculer le couple de charge estimé $T_{load}$ au niveau de la fraise d'athérectomie (14) selon l'équation (1) :

$$T_{load} = K_T * i - C_D * \dot{\theta} - I * \ddot{\theta} \qquad (1),$$

où

$K_T$ est une constante de couple pour le moteur d'entraînement ;
$i$ est un courant du moteur d'entraînement ;
$C_D$ est un coefficient de valeur de frottement ;
$\dot{\theta}$ est la vitesse angulaire du système d'athérectomie ;
$I$ est une inertie du système d'athérectomie ; et
$\ddot{\theta}$ est l'accélération angulaire du système d'athérectomie.

6.  Système d'athérectomie (10 ; 20 ; 40 ; 50 ; 300) selon la revendication 5, dans lequel $i$ est une valeur mesurée ou calculée.

**7.** Système d'athérectomie (10 ; 20 ; 40 ; 50 ; 300) selon la revendication 5, dans lequel $C_D$ est une constante.

**8.** Système d'athérectomie (10 ; 20 ; 40 ; 50 ; 300) selon la revendication 5, dans lequel $C_D$ est une valeur calculée.

**9.** Système d'athérectomie (10 ; 20 ; 40 ; 50 ; 300) selon l'une quelconque des revendications 1 à 8, dans lequel le mécanisme d'entraînement (12) comprend :

un câble d'entraînement (24 ; 304) couplé à la fraise d'athérectomie (14) ; et
un moteur d'entraînement (22 ; 302) adapté pour faire tourner le câble d'entraînement (24 ; 304).

**10.** Système d'athérectomie (10 ; 20 ; 40 ; 50 ; 300) selon la revendication 1,

dans lequel le dispositif de commande (16 ; 42 ; 52 ; 350) est adapté pour calculer un couple de charge estimé au niveau de la fraise d'athérectomie $T_{load}$ selon l'équation (2) :

$$T_{load} = T_{motor} - T_{drag} - I * \ddot{\theta} \qquad (2),$$

où
$T_{motor}$ est un couple de moteur estimé pour le moteur d'entraînement ;
$T_{drag}$ est un couple de traînée estimé pour le mécanisme d'entraînement ;
$I$ est une valeur d'inertie du système ; et
$\ddot{\theta}$ est une valeur d'accélération angulaire.

**11.** Système d'athérectomie (10 ; 20 ; 40 ; 50 ; 300) selon la revendication 10, dans lequel $T_{motor}$ est calculé par le dispositif de commande (16 ; 42 ; 52 ; 350) selon l'équation (3) :

$$T_{motor} = K_T * i \qquad (3),$$

où

$K_T$ est une constante de couple pour le moteur d'entraînement ; et
$i$ est un courant de moteur d'entraînement.

**12.** Système d'athérectomie (10 ; 20 ; 40 ; 50 ; 300) selon la revendication 11, dans lequel i est une valeur mesurée ou calculée.

**13.** Système d'athérectomie (10 ; 20 ; 40 ; 50 ; 300) selon la revendication 10, dans lequel

$T_{drag}$ est calculé par le dispositif de commande (16 ; 42 ; 52 ; 350) selon l'équation (4) :

$$T_{drag} = C_D * \dot{\theta} \qquad (4),$$

où
$C_D$ est un coefficient de valeur de frottement ; et
$\dot{\theta}$ est une valeur de vitesse angulaire.

**14.** Système d'athérectomie (10 ; 20 ; 40 ; 50 ; 300) selon la revendication 10, dans lequel le mécanisme d'entraînement (12) comprend :

un câble d'entraînement (24 ; 304) couplé à la fraise d'athérectomie (14) ; et
un moteur d'entraînement (22 ; 302) adapté pour faire tourner le câble d'entraînement (24 ; 304).

**15.** Système d'athérectomie (10 ; 20 ; 40 ; 50 ; 300) selon la revendication 1,

dans lequel, lorsque le système d'athérectomie (10 ; 20 ; 40 ; 50 ; 300) se trouve dans un état stationnaire, le dispositif de commande (16 ; 42 ; 52 ; 350) est adapté pour calculer la valeur de couple estimé $T_{load}$ selon l'équation (5) :

$$T_{load} = -I * \ddot{\theta} \qquad (5),$$

où

$I$ est une inertie du système d'athérectomie ; et

$\ddot{\theta}$ est l'accélération angulaire du système d'athérectomie ; et

dans lequel, lorsque le système d'athérectomie (10 ; 20 ; 40 ; 50 ; 300) accélère, le dispositif de commande (16 ; 42 ; 52 ; 350) est adapté pour calculer $T_{load}$ selon l'équation (1) :

$$T_{load} = K_T * i - C_D * \dot{\theta} - I * \ddot{\theta} \qquad (1).$$

où

$K_T$ est une constante de couple pour le moteur d'entraînement ;

$i$ est un courant du moteur d'entraînement ;

$C_D$ est un coefficient de valeur de frottement ;

$\dot{\theta}$ est la vitesse angulaire du système d'athérectomie (10 ; 20 ; 40 ; 50 ; 300).

FIG. 1

EP 3 952 763 B1

FIG. 2

*40*

*42*

*32* *46* *44* *48* *12* *14*

| Reference Block | Speed ref | PID Controller | Output Signal | Drive Mechanism | Burr |

Control System

FIG. 3

EP 3 952 763 B1

FIG. 4

FIG. 5

FIG. 6

EP 3 952 763 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62830990
- US 4679557 A